Europäisches Patentamt

European Patent Office

Office européen des brevets

(11) Publication number: **0 473 101 A1**

# EUROPEAN PATENT APPLICATION

(21) Application number: **91114319.6**

(22) Date of filing: **27.08.91**

(51) Int. Cl.5: **G01N 33/52**, C12Q 1/32

(30) Priority: **29.08.90 JP 228798/90**

(43) Date of publication of application:
**04.03.92 Bulletin 92/10**

(84) Designated Contracting States:
**CH DE FR GB IT LI**

(71) Applicant: **KYOTO DAIICHI KAGAKU CO., LTD.**
**57 Nishiaketa-cho Higashikujo Minami-ku**
**Kyoto-shi Kyoto-fu(JP)**

(72) Inventor: **Sakamoto, Hisashi**
**7-E-16-202, Yutoku, Otokoyama**
**Yawata-shi, Kyoto-fu(JP)**
Inventor: **Nakano, Hajime**
**74, Masuya-cho, Takakuranishiiru,**
**Hanayachodori**
**Shimogyo-ku, Kyoto-shi, Kyoto-fu(JP)**

(74) Representative: **von Kreisler, Alek,**
**Dipl.-Chem. et al**
**Patentanwälte Von Kreisler-Selting-Werner,**
**Deichmannhaus am Hauptbahnhof**
**W-5000 Köln 1(DE)**

(54) **Integral multilayer analytical element and preparation thereof.**

(57) An integral multilayer analytical element having a tetrazolium salt-containing layer, an alkaline buffer-containing layer and an intermediate layer which is interposed between said tetrazolium salt-containing layer and the alkaline buffer-containing layer, wherein the tetrazolium salt-containing layer and the alkaline buffer-containing layer are laminated after applying a solvent in which at least one of these two layers is not dissolved on a surface to be laminated of at least one of these two layers, which element is stable and easily prepared.

EP 0 473 101 A1

## BACKGROUND OF THE INVENTION

### Field of the Invention

The present invention relates to an integral multilayer analytical element for analyzing one or more components in a body fluid and a process for preparing the same. More particularly, the present invention relates to an integral multilayer analytical element comprising one layer containing a tetrazolium salt and another layer containing an alkaline buffer and its preparation.

### Description of the Related Art

In the analysis of a component in a body fluid, a certain pair of a substrate and a color reagent will be unstabilized and gradually colored when they exist together. In particular, when a tetrazolium salt is present together with an alkaline buffer, it is strongly colored as time passes. Then, various methods have been proposed to separate the tetrazolium salt from the alkaline buffer in the analytical element.

U.S. Patent No. 4,351,899 (to Owen et al) discloses the use of an analytical element in the analysis of a component in a body fluid, which element is prepared by impregnating a first filter paper with a reagent solution and drying it to form a reagent layer, separately impregnating a second filter paper with an alkaline buffer and drying it to form a buffer-containing layer, and physically laminating these two layers to form an analytical element. The method of Owen et al can separate the tetrazolium salt, an enzyme or a coenzyme from the alkaline buffer completely, but requires a tool (e.g. a mount, etc.) for laminating the two layers for measurement. In case of mass production of the analytical elements of Owen et al, a processing machine for mounting and laminating the two layers should be introduced, which results in great increase of a production cost.

Japanese Patent Kokai Publication No. 94995/1988 and corresponding EP-A-264 019 disclose a method for producing an analytical element comprising dissolving or dispersing a buffer which is soluble in an organic solvent in a solvent, spraying or impregnating the solution or suspension in a porous developing layer and laminating the developing layer on a reagent layer. The use of the alkaline buffer which is soluble in the organic solvent and also in water of a sample greatly limits flexibility of selection of a buffer having a good buffer capacity, and the number of the alkaline buffers having such solubility is small. In addition, it is extremely difficult to measure pH of the solution in the organic solvent precisely, and a salt may be formed and precipitated when a base or an acid is added to the solution for adjusting pH, whereby pH becomes less accurate. It will be practically impossible to uniformly spray or impregnate the solution of the buffer in the organic solvent containing the precipitated salt.

## SUMMARY OF THE INVENTION

One object of the present invention is to provide an integral multilayer analytical element which is stable and easily prepared.

Another object of the present invention is to provide an integral multilayer analytical element which contains a tetrazolium salt and an alkaline buffer in discrete layers.

A further object of the present invention is to provide a process for preparing an integral multilayer analytical element which contains a tetrazolium salt and an alkaline buffer in discrete layers.

According to a first aspect of the present invention, there is provided an integral multilayer analytical element comprising a tetrazolium salt-containing layer, an alkaline buffer-containing layer and an intermediate layer which is interposed between said tetrazolium salt-containing layer and said alkaline buffer-containing layer, wherein said tetrazolium salt-containing layer and said alkaline buffer-containing layer are laminated after applying a solvent in which at least one of said two layers is not dissolved on a surface to be laminated of at least one of said two layers.

According to a second aspect of the present invention, there is provided a process for preparing an integral multilayer analytical element, which comprises steps of

providing a tetrazolium salt-containing layer and an alkaline buffer-containing layer,

applying a solvent in which at least one of said two layers is not dissolved on a surface to be laminated of at least one of said two layers,

laminating said two layers, and

drying laminated layers to remove said solvent.

## DETAILED DESCRIPTION OF THE INVENTION

The tetrazolium salt to be used in the present invention may be any one of conventional tetrazolium salts. Specific examples of the tetrazolium salt are:

3,3'-[3,3'-dimethoxy-(1,1'-biphenyl)-4,4'-diyl]-bis[2-(p-nitrophenyl)-5-phenyl-2H-tetrazolium chloride] (NTB),

3,3'-[3,3'-dimethoxy-(1,1'-biphenyl)-4,4'-diyl]-bis[2,5-bis(p-nitrophenyl)-2H-tetrazolium chloride] (TNTB),

3,3'-(1,1'-biphneyl-4,4'-diyl)-bis(2,5-diphenyl-2H-tetrazolium chloride] (NeoTB),

3,3'-[3,3'-dimethoxy-(1,1'-biphenyl)-4,4'-diyl]-bis(2,5-diphenyl-2H-tetrazolium chloride) (TB),

3-(p-iodophenyl)-2-(p-nitrophenyl)-5-phenyl-2H-tetrazolium chloride (INT),

3-(4,5-dimethyl-2-thiazolyl)-2,5-diphenyl-2H-tetrazolium bromide (MTT), and

2,5-diphenyl-3-(1-naphthyl)-2H-tetrazolium chloride (TV).

The alkaline buffer may be any one of conventional buffers having a buffering capacity at pH of larger than 7.

The buffer-containing layer may contain at least one other reagent according to a compound in the body fluid to be analyzed. Examples of such reagent are as follows:

Lactic acid: lactic dehydrogenase, NAP, diaphorase.

Glucose: glucose dehydrogenase, NAD, diaphorase.

Ketone body: 3-hydroxybutyric dehydrogenase, NAD, diaphorase.

Cholesterol: cholesterol dehydrogenase, NAD, diaphorase.

Bile acid: 3$\alpha$-hydroxysteroid dehydrogenase, NAD, diaphorase.

Alcohol: alcohol dehydrogenase, NAD, diaphorase.

When fructosamine is to be analyzed, no other reagent is required.

The integral multilayer analytical of the present invention may be prepared by forming the tetrasolium salt-containing layer and the alkaline buffer-containing layer, applying a solvent in which at least one of these two layers is not dissolved on a surface to be laminated of at least one of these two layers, laminating the two layers, and drying the laminated layers to remove the solvent.

The alkaline buffer-containing layer may be formed by dissolving the alkaline buffer and optionally a binder in a suitable solvent, applying a resulting solution on a suitable substrate, and drying it. Preferably, the binder is a hydrophilic polymer such as polyvinylpyrrolidone, hydroxypropylcellulose, methylcellulose, sodium alginate, polyacrylic acid, gelatin, etc. When the hydrophilic polymer is used, the solvent is an aqueous solvent, in particular, water.

A concentration of the alkaline buffer is not critical. When a high concentration solution is used, the layer is formed by one application of the solution, while a low concentration solution is used, the layer is formed by plural applications of the solution.

As the substrate, any of the conventionally used ones (e.g. polymer films, glass, metals, etc.) can be used.

The tetrazolium salt-containing layer may be formed by impregnating a porous matrix (e.g. a filter paper, a fabric, a membrane filter, etc.) with a solution of the tetrazolium salt and drying it. A solvent is selected according to a kind of the tetrazolium salt. A solvent in which a specific tetrazolium salt is dissolved is well known.

A concentration of the tetrazolium salt is not critical. When a high concentration solution is used, the layer is formed by one impregnation of the solution, while a low concentration solution is used, the layer is formed by plural impregnations of the solution.

A solvent to be used for forming the intermediate layer is selected from solvents in which at least one of the tetrazolium salt-containing layer and the alkaline buffer-containing layer is not dissolved, preferably neither of them is dissolved. Examples of the solvent are acetone, isopropanol, n-propanol, butanol, chloroform, methylene chloride, toluene, or mixtures thereof.

Since at least one of these two layers is not dissolved in the solvent, when the solvent is applied on at least one of the tetrazolium salt-containing layer and the alkaline buffer-containing layer and the layers are laminated, the tetrazolium salt and the alkaline buffer are not mixed in the intermediate layer, so that the tetrazolium salt and the buffer are separated in the multilayer analytical element of the present invention.

The solvent used for forming the intermediated layer may contain a binder resin which is soluble in the solvent, in particular, when neither layer is dissolved in the solvent. The binder may be the same as exemplified above in connection with the formation of the alkaline buffer-containing layer.

## PREFERRED EMBODIMENTS OF THE INVENTION

The present invention will be illustrated by following Examples.

Example

Preparation of a multilayer element for analyzing lactic acid

To form a reagent/alkaline buffer-containing layer, the following components were mixed, coated on an undercoated polyethylene terephthalate film and dried at 40°C for 30 minutes:

| | |
|---|---|
| Lactic dehydrogenase | 30 KU |
| β-NAD | 100 mg |
| Diaphorase | 5 KU |
| 0.3 M carbonate buffer (pH 9.0) | 9 g |
| Polyvinylpyrrolidone | 1 g |
| 10 % Triton (trade mark) X-100 | 50 mg |

To form a tetrazolium (color reagent)-containing layer, the following components were mixed, impregnated in a porous matrix (knitted synthetic fibers) and dried at 40°C for 30 minutes:

| | |
|---|---|
| NTB | 300 mg |
| Methanol | 10 g |
| Water | 14.7 g |

Then, hydroxypropylcellulose (2 g) and isopropanol (98 g) were mixed and coated on the porous matrix containing the impregnated tetrazolium salt by, for example, gravure coating. Then, the porous matrix was laminated on the alkaline buffer-containing layer and dried at 40°C for 30 minutes to obtain an integral laminated article.

This article was cut to a size of 5 mm x 7 mm and fixed at one end of a base film (5 mm x 80 mm) with a double-coated tape to obtain a sample element.

Comparative Example

In the same manner as in Example but using purified water in place of isopropanol, a comparative sample element was prepared.

Evaluation (I) of the sample elements

A background of the sample element prepared in each of Example and Comparative Example was measured in terms of a reflectance at a wavelength of 550 nm, and the measured reflectance was converted to a K/S value according to the Kubelka-Munk equation.

The sample elements of Example and Comparative Example had the K/S values of 0.05 and 1.24, respectively. This means that the background of the element of Example was less than one twentieth of that of the element of Comparative Example.

Since coloring of the background is suppressed, an optical dynamic range is guaranteed and the element is stably produced according to the present invention.

Evaluation (2) of the sample elements

On each sample element, an aqueous solution of lactic acid in various concentrations was spotted and reflectance was measured after 3 minutes to draw a calibration line.

With each of the aqueous solutions of lactic acid having concentrations of 20 mg/dl and 80 mg/dl, the reflectance was measured with each of the elements of Example and Comparative Example ten times and simultaneous reproducibility was compared by converting the reflectance to the concentration using the calibration line. The results are as follows.

| Run No. | Example | | Comp. Example | |
|---|---|---|---|---|
| | 20 mg/dl | 80 mg/dl | 20 mg/dl | 80 mg/dl |
| 1 | 20.5 | 81.8 | 21.4 | 80.5 |
| 2 | 19.8 | 83.8 | 22.7 | 83.4 |
| 3 | 21.0 | 81.5 | 18.5 | 81.5 |
| 4 | 20.3 | 80.3 | 19.7 | 78.3 |
| 5 | 20.7 | 81.7 | 22.5 | 79.8 |
| 6 | 19.7 | 78.6 | 21.9 | 84.2 |
| 7 | 21.2 | 82.1 | 19.2 | 85.0 |
| 8 | 20.3 | 79.8 | 20.6 | 80.1 |
| 9 | 20.9 | 81.4 | 21.8 | 77.4 |
| 10 | 20.4 | 84.2 | 20.8 | 82.8 |
| $\bar{x}$ | 20.48 | 81.71 | 20.91 | 81.3 |
| S.D. | 0.49 | 1.63 | 1.42 | 2.52 |
| C.V. | 2.4 | 2.0 | 6.8 | 3.1 |

The simultaneous reproducibility of the element of Example was in the order of 2 %, while that of the element of Comparative Example varied from 3 % to 7 %. This may be because color of the colored parts which act as the background might have differ from part to part and then the deviation became large. The present invention improves the reliability greatly.

**Claims**

1.  An integral multilayer analytical element comprising a tetrazolium salt-containing layer, an alkaline buffer-containing layer and an intermediate layer which is interposed between said tetrazolium salt-containing layer and said alkaline buffer-containing layer, wherein said tetrazolium salt-containing layer and said alkaline buffer-containing layer are laminated after applying a solvent in which at least one of said two layers is not dissolved on a surface to be laminated of at least one of said two layers.

2.  The integral multilayer analytical element according to claim 1, wherein said solvent contains a binder, and said intermediate layer consists of said binder.

3.  The integral multilayer analytical element according to claim 1, wherein said binder is a hydrophilic polymer and said solvent is at least one selected from the group consisting of acetone, isopropanol, n-propanol, butanol, chloroform, methylene chloride and toluene.

4.  The integral multilayer analytical element according to claim 3, wherein said hydrophilic polymer is selected from the group consisting of polyvinylpyrrolidone, hydroxypropylcellulose, methylcellulose, sodium alginate, polyacrylic acid and gelatin.

5.  A process for preparing an integral multilayer analytical element, which comprises steps of
    providing a tetrazolium salt-containing layer and an alkaline buffer-containing layer,

applying a solvent in which at least one of said two layers is not dissolved on a surface to be laminated of at least one of said two layers,

laminating said two layers, and

drying laminated layers to remove said solvent.

6. The process according to claim 5, wherein said solvent contains a binder, and said intermediate layer consists of said binder.

7. The process according to claim 5, wherein said binder is a hydrophilic polymer and said solvent is at least one selected from the group consisting of acetone, isopropanol, n-propanol, butanol, chloroform, methylene chloride and toluene.

8. The process according to claim 7, wherein said hydrophilic polymer is selected from the group consisting of polyvinylpyrrolidone, hydroxypropylcellulose, methylcellulose, sodium alginate, polyacrylic acid and gelatin.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,D | EP-A-0 264 079   (FUJI PHOTO FILM CO., LTD.)<br>* PAGES 1-8; PAGE 13, LINE 25- PAGE 14, LINE 20, CLAIMS 1-11.* * | 1-8 | G 01 N 33/52<br>C 12 Q 1/32 |
| X | US-A-4 939 085   (A. FUMINORI)<br>* the whole document * * | 1-8 | |
| X | EP-A-0 116 361   (FUJI PHOTO FILM CO., LTD.)<br>* PAGES 1-14; EXAMPLE 2; CLAIMS 1-9.* * | 1,2,4-6,8 | |
| Y | EP-A-0 205 950   (FUJI PHOTO FILM CO., LTD.)<br>* the whole document * * | 1-8 | |
| Y | PATENT ABSTRACTS OF JAPAN vol. 12, no. 372 (C-533)(3219) 5 October 1988<br>& JP-A-63 119 694 ( FUJI PHOTO FILM CO., LTD. ) 24 May 1988<br>* abstract * * | 1-8 | |
| | | | **TECHNICAL FIELDS SEARCHED (Int. Cl.5)**<br><br>G 01 N<br>C 12 Q |

The present search report has been drawn up for all claims

| Place of search | Date of completion of search | Examiner |
|---|---|---|
| The Hague | 27 November 91 | HITCHEN C.E. |